# EUROPEAN PATENT APPLICATION

(11) **EP 4 257 158 A1**
(43) Date of publication of application: **11.10.2023**
(21) Application number: 22382327.9
(22) Date of filing: 06.04.2022
(51) Int. Cl.: A61L 27/38, A61L 27/52, A61L 27/60, A61F 2/10, C12N 5/0735, C12N 5/074, C12N 5/071

(54) **BIOMIMETIC MODEL OF NON-MELANOMA SKIN CANCER AND USES THEREOF**

(71) Applicant: Servicio Andaluz de Salud, 41071 Sevilla (ES); Universidad de Granada, 18071 Granada (ES)
(72) Inventor: ALAMINOS MINGORANCE, Miguel, 18071 Granada (ES); CARRIEL ARAYA, Víctor Sebastián, 18071 Granada (ES); CHATO ASTRAIN, Jesús, 18071 Granada (ES); CAMPOS MUÑOZ, Antonio, 18071 Granada (ES); FERNÁNDEZ VALADÉS, Ricardo, 41071 Sevilla (ES)
(74) Representative: Hoffmann Eitle

(57) **Abstract**

The present invention relates to a method for the production of a biomimetic model of non-melanoma skin cancer comprising a hydrogel and at least a cell population comprising squamous carcinoma keratinocyte cells. Uses of said skin model are also provided.

## Description

### TECHNICAL FIELD

The present invention relates to the field of biomedicine. Particularly, the present invention relates to the field of tissue engineering, more specifically to the field of biomaterials and skin models.

### BACKGROUND ART

Tissue engineering is a promising and interdisciplinary active area of research in biomedical engineering that provides and investigates the application of novel biomaterials for the reconstruction of diseased or damaged tissues and organs. Several important characteristic factors such as tunable physical, morphological and mechanical properties, suitable permeability, biocompatibility, non-toxicity and non-inflammatory; among others, need to be carefully considered in the fabrication of a functional skin substitute. Further, to produce bioartificial tissues or substitutes for tissue engineering, it is necessary to combine the methods and principles of medicine, biology, chemistry and engineering accompanied by adequate quality controls. In this sense, for the development of artificial tissues the presence and close interrelation of three fundamental elements is necessary: cells, biomaterials and growth factors. All these elements, in turn, represent a range of possibilities according to the different characteristics that they can offer.

Scaffolds are the backbones of any tissue-engineered skin substitute. They provide a platform for cells during the growth process. The structure, morphology, surface topography and mechanical elasticity of scaffolds play a crucial role in cell metabolic activities (e.g., cell-adhesion, -proliferation, -growth, and -differentiation) for successful neovascularization and complete wound repair. Traditional methods such as solvent casting/particulate leaching, freeze-drying (lyophilization), gas foaming, electrospinning, micro-patterning and micro-molding have been widely used for the fabrication of bioengineered tissue substitutes. Recently, advanced biofabrication strategies such as three-dimensional (3D) bioprinting have emerged as powerful tools that enable exquisite control over the micro and cytostructure of the bioengineering skin tissues. Hydrogels also represent an attractive scaffold for tissue engineering, particularly those based on fibrin and agarose. See patent ES2362139 for an example of a fibrine-agarose hydrogel developed for tissue engineering.

Another important component of tissue engineering are the cells. To build an artificial tissue, it is essential to know the fundamental principles on which living systems are created, whose basic functional and structural component are cells. It is therefore necessary to first know the strategies that cells use to regenerate and build new tissue under normal conditions. In this complex process, different biological behaviours occur where we can recognize two circumstances in which many of the cellular potentialities develop, specifically normal morphogenetic development and wound healing.

The skin is the most common substrate for malignant neoplasms in humans. The combined lifetime risk of all skin cancers is estimated to be 30-40% for lightly pigmented (Northwest) Europeans, although this value depends on individual skin pigmentation. From a histological point of view, there are three fundamental types of skin cancer: melanoma, squamous cell carcinoma (SCC) and basal cell carcinoma (BCC). The first type of cancer is generated from skin melanocytes, while the other two, which together constitute the so-called NMC, originate by transformation of epidermal keratinocytes. Of these, SCC rarely metastasizes and BBC rarely. Although the vast majority of NMC tumours are detected in early and well-localized stages (grades I and II), SCC is responsible for the majority of NMC deaths and accounts for approximately 20% of all NMC-related deaths skin cancer.

Tissue engineered skin substitutes provide new therapy potentials for screening new drugs against skin cancers. Further, tissue engineering is also important to comply with Replacement, Refinement and Reduction of Animals in Research/Biotechnology, as they represent valid alternatives to test new drugs and to study the behaviour or nature of skin cancers. The present invention aims to provide a biomimetic skin model of non-melanoma skin cancer and uses thereof.

### SUMMARY OF INVENTION

In a first aspect, the present invention provides a method for the *in vitro* production of a non-melanoma skin cancer model, preferably a biomimetic squamous cell carcinoma skin model, the method comprising the steps of:
a) providing a hydrogel comprising agarose and fibrin,
b) adding to the hydrogel of step a), preferably on its surface, a cell population comprising squamous carcinoma keratinocyte cells,
c) optionally, prior to, simultaneously to, or subsequently to step b), adding to the hydrogel, preferably on its surface, a cell population comprising non-cancerous keratinocyte cells, and/or a cell population comprising mesenchymal cells, and
d) culturing the cells of the product resulting from (b) or, optionally, the product resulting from (c), in a suitable culture medium to allow the cells to grow and expand to provide a biomimetic squamous cell carcinoma skin model.

Preferably, the preparation of a hydrogel according to step a) comprises the steps of:
i. preparing a polymer solution comprising agarose, wherein the polymer solution is maintained in a liquid state,
ii. adding to the polymer solution of i), in any order, an antifibrinolytic agent, a source of calcium, fibrin or a source of fibrin, a source of clotting factor of type II and, optionally, a cell population comprising stromal cells, and
iii. allowing the resulting solution of step ii) to polymerize, preferably at 37°C.

Preferably, the cell population comprising stromal cells is added in step ii), and said stromal cells are preferably fibroblasts. Preferably, the agarose comprised in the polymer solution of step i) is at a final concentration of between 0.05 and 0.6% (w/v), preferably 0.1%. Preferably, the clotting factor of type II added in step ii) is thrombin or prothrombin. Preferably, the source of clotting factor of type II and the source of fibrin added in step ii) is blood plasma, and the blood plasma added is at a final concentration of between 10% and 95% (v/v). Preferably, the antifibrinolytic agent added in step ii) is at a final concentration of between 0.5% and 3%, preferably 1.5% (w/v), and it is preferably tranexamic acid. Preferably, the source of calcium added in step ii) is CaCl₂. Preferably, the population comprising non-cancerous keratinocytes cells of step c) are added and is preferably a primary culture of non-cancerous human keratinocytes. Preferably, the total number of cells added to the hydrogel is of about 1000 to 1 million of cells per millilitre of hydrogel. Preferably, the step d) is performed by first completely submerging the skin model in the suitable medium for at least 2 days and then culturing the cells comprised in the skin model in an air-liquid interface system for at least 10 days, preferably 14 days.

In a second aspect, the present invention provides a non-melanoma skin cancer model, preferably a biomimetic squamous cell carcinoma skin model, obtained or obtainable from the method according to the first aspect or any of its embodiment.

In another aspect, the present invention provides the in vitro use of the non-melanoma skin cancer model, preferably the biomimetic squamous cell carcinoma skin model, obtained or obtainable from the method according to the first aspect or any of its embodiments, in the pharmacological development and preclinical screening of chemotherapeutic drugs, and basic study of the fundamental biology of cancer and the processes that mediate tumor proliferation, growth, metastasis and invasion.

### BRIEF DESCRIPTION OF DRAWINGS

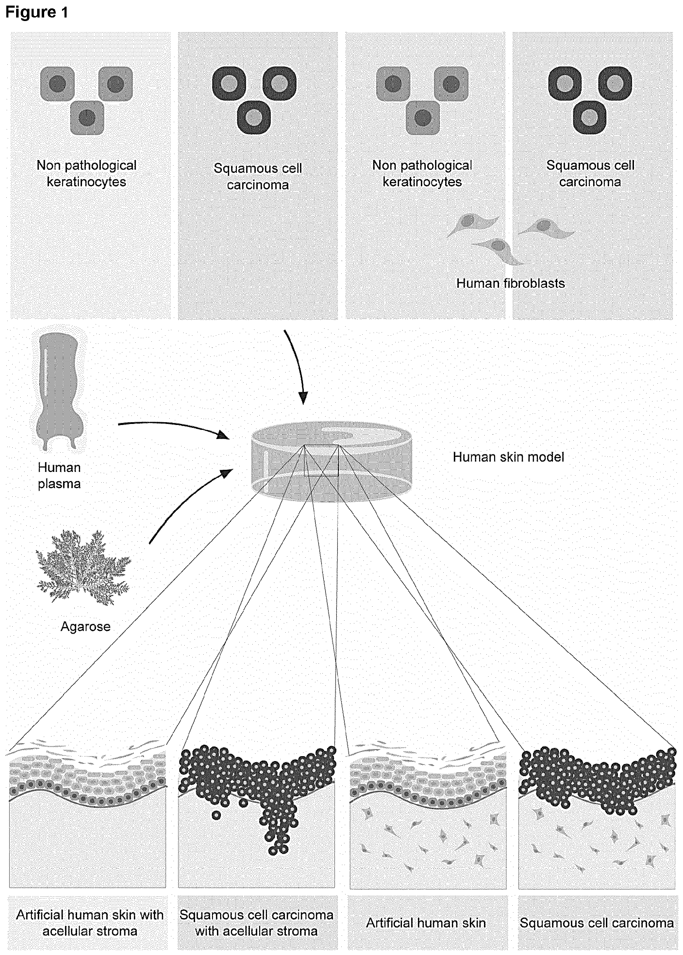

**Fig. 1****: Carcinoma and normal human skin models biofabrication.** A fibrin-agarose hydrogel was used in all pathological and normal models combining different cell sources. SCC-derived cells and non-pathological keratinocytes were seeded on top of the hydrogel to generate the acellular stroma of the pathological and normal model respectively. For the cellular stroma models, human fibroblasts were also included into the HFA.

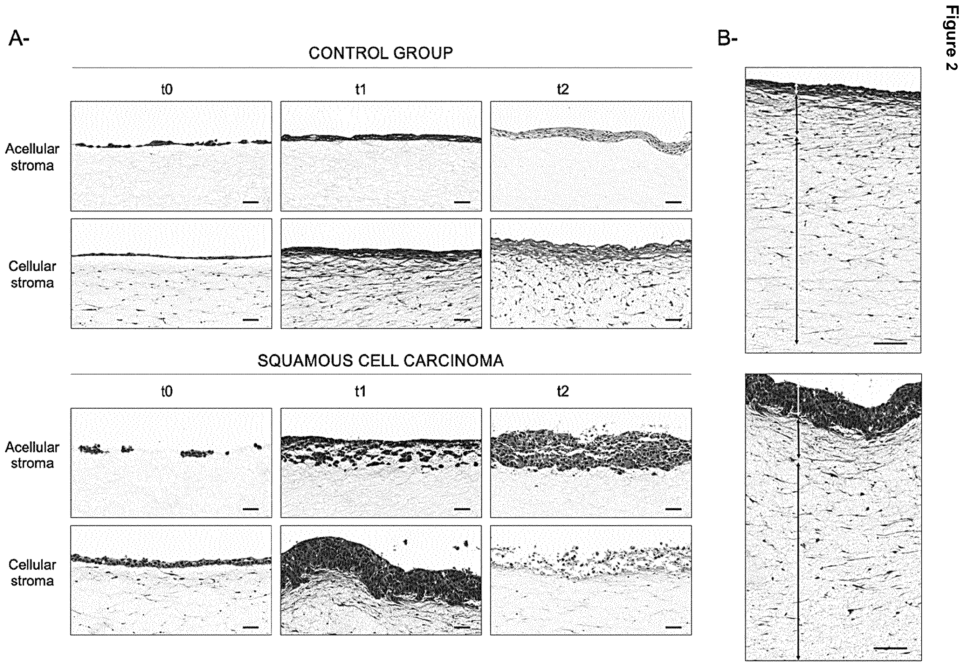

**Fig. 2****: Histological assessment of the carcinoma and normal human skin models.** Hematoxylin-eosin study of the tissue engineered models. A-Epidermis study of pathological and no pathological models. Cellular and acellular stroma were displayed in the upper and lower part respectively. Scale bar = 50 µm. B-Celular dermis study of the pathological and no pathological model. Scale bar = 25 µm

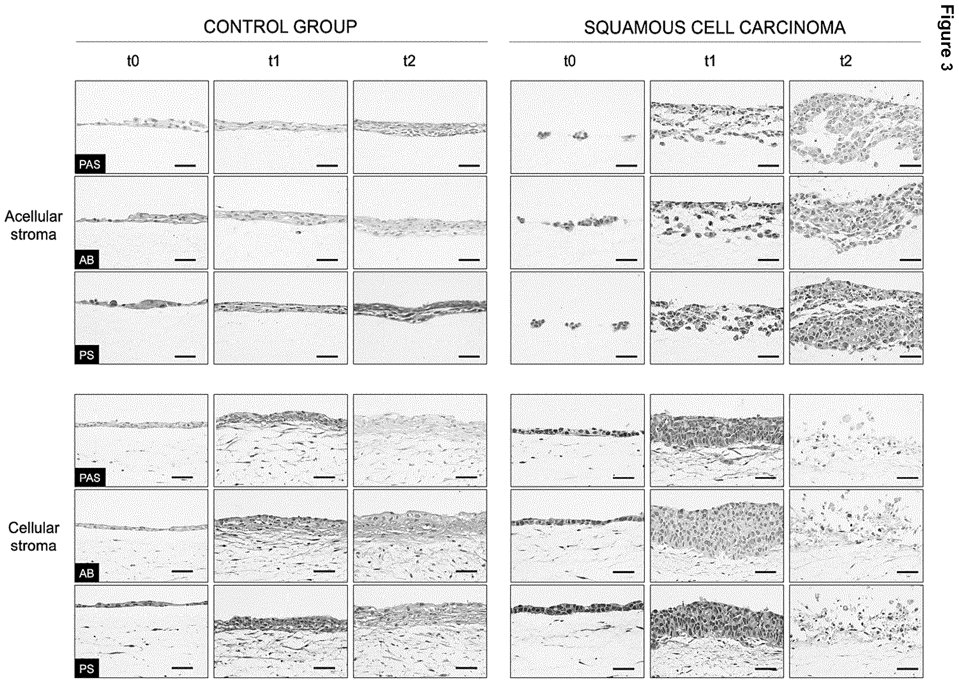

**Fig. 3****: Extracellular matrix evaluation of the carcinoma and normal human skin models.** Cellular and acellular stroma were displayed in the upper and lower part respectively. All samples were stained with periodic acid of Schiff (PAS), alcian blue (AB) and picrosirius red (PS) to evaluate the presence and spatial organization of carbohydrates, proteoglycans and collagen fibers respectively. Scale bar = 50 µm

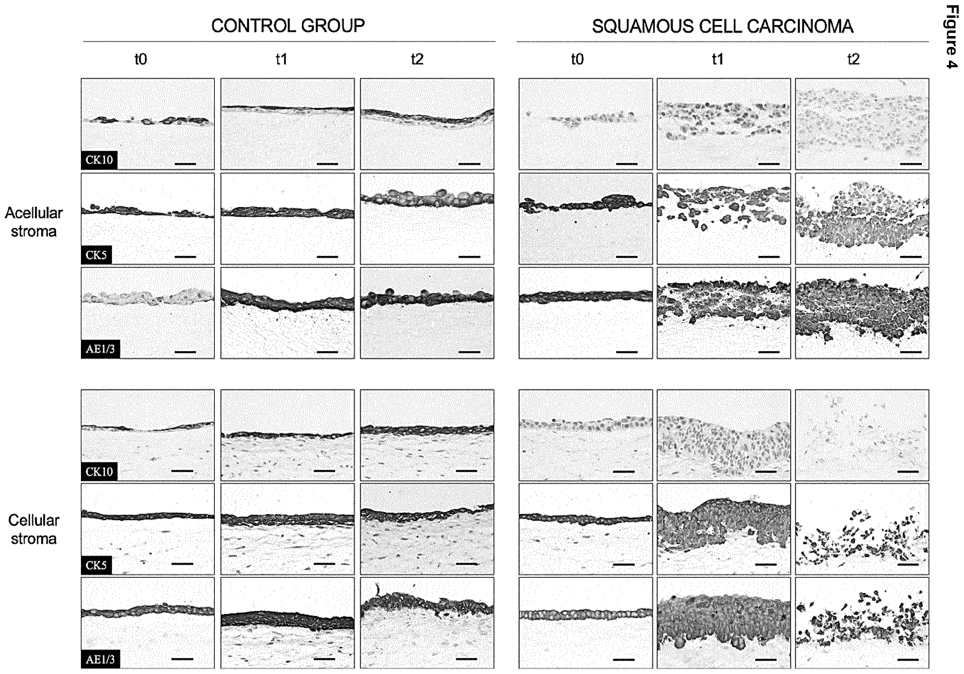

**Fig. 4****: Epidermis characterization of the carcinoma and normal human skin models.** Immunohistochemical detection of cytokeratin 10 (CK10), cytokeratin 5 (CK5) and cytokeratin AE1/3 (CKAE1/3). Cellular and acellular stroma were displayed in the upper and lower part respectively. Scale bar = 50 µm

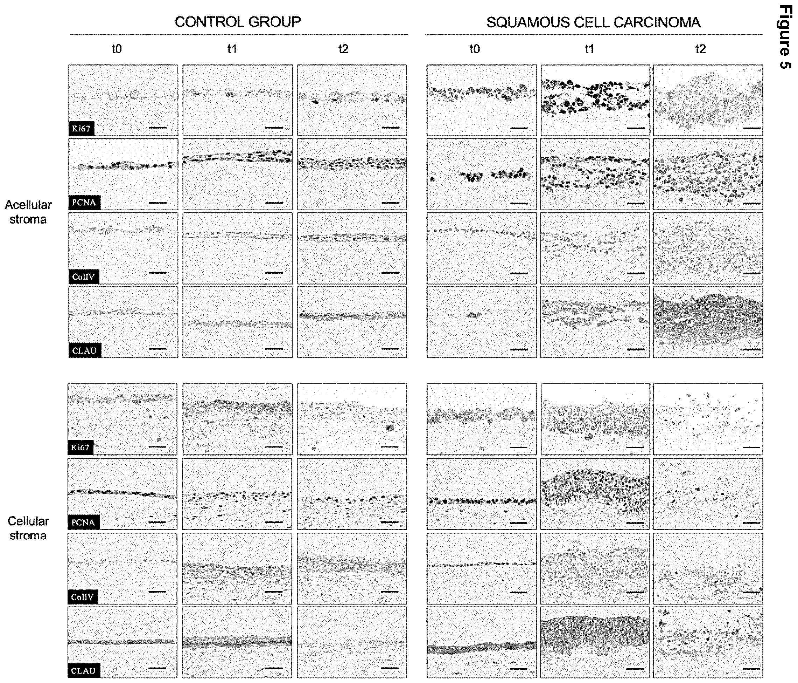

**Fig. 5****: Immunohistochemical assessment of the carcinoma and normal human skin models.** Immunodetection of cell proliferation associated proteins (Ki67 and PCNA), basal membrane components (collagen IV - ColIV) and intercellular junction protein (claudin - CLAU). Cellular and acellular stroma were displayed in the upper and lower part respectively. Scale bar = 50 µm

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

As used herein "autologous" is understood as referring to a cell preparation where the donor and the recipient are the same individual. As used herein "allogeneic" or "xenogenic" is understood as referring to a cell preparation where the donor and the recipient are not the same individual.

The term "cell population" refers to a group of cells. A cell population is heterogeneous when it comprises different groups of cells, wherein each group is differentiated from others by the presence of one or more distinguishing characteristics, such as the expression or not expression of specific markers or the presence of a different function.

### DESCRIPTION OF THE EMBODIMENTS

Tissue engineering is one of the areas of biotechnology most developed in recent years because of its usefulness for *in vitro* production of tissues and organs for implantation in patients in need of these tissues However, the limitations of existing artificial tissues so far require the development of new techniques that will produce artificial tissues that can be used in human clinical or assessment of pharmaceutical products and chemicals. Several types of skin cancer fall within the broader category of non-melanoma skin cancer, with the most common types being basal cell carcinoma (BCC) and squamous cell carcinoma (SCC).

The main objective of the present invention was the generation of new biomimetic models of non-melanoma SCC skin cancer model, through the use of fibrin-agarose hydrogels, with the objective of providing an in vitro model suitable for drug discovery and research in the field of non-melanoma skin cancers. With this purpose, several skin models comprising cellular and acellular stroma were generated, as shown in Fig. 1, and they were structurally and functionally studied. The results showed that the models of SCC (with and without cellular stroma) based on fibrin-agarose hydrogels were structurally highly biomimetic with respect to native human skin controls. The histological analysis carried out in the SCC models showed characteristics of tumor dissemination, invasion of tumor cells in the stroma and spatial disorganization, all of which is compatible with an orthotypic SSC. The study of the differentiation and maturation markers of human skin showed that the model is capable of reproducing the main components of the extracellular matrix of normal skin, especially in the non-melanoma skin cancer model with cellular stroma, as well as the main biomarkers of neoplastic epithelium, with poorly differentiated keratinocytes and high proliferation capacity (Figures 4 and 5).

Consequently, the non-melanoma skin cancer models generated in this study showed great potential for the study of the pathophysiology of this type of cancer, as well as for the ex *vivo* evaluation of antitumor agents and new therapeutic protocols.

In view of the above, in a **first aspect,** the present invention relates to a method, preferably *in vitro,* for generating a non-melanoma skin cancer model, preferably a biomimetic squamous cell carcinoma skin model, comprising the steps of:
a) providing a hydrogel comprising at least one polysaccharide, preferably agarose, and fibrin,
b) adding to the hydrogel of step a), preferably on its surface, a cell population comprising squamous carcinoma keratinocyte cells,
c) optionally, prior to, simultaneously to, or subsequently to step b), adding to the hydrogel, preferably on its surface, a cell population comprising non-cancerous keratinocyte cells, and/or a cell population comprising mesenchymal cells, and
d) culturing the cells of the product resulting from (b) or, optionally, the product resulting from (c) in a suitable culture medium to allow the cells to grow and expand and/or to differentiate to provide a biomimetic squamous cell carcinoma skin model.

By adding to the hydrogel of step a) the cells of step b) or, optionally, the cells of step c), and after leaving to stand the resultant product of step d), the cultured cells are partially embedded and on which and/or inside the hydrogel where they can grow. Preferably, the cells of steps b) and c) are added on the surface of the hydrogel of step a), but eventually grow inside of said matrix.

### Step a) providing a hydrogel comprising at least one polysaccharide:

Step a) of the method of the first aspect or any of its embodiments comprises providing a hydrogel comprising at least one polysaccharide, preferably agarose, and fibrin or a source of fibrin. The term "hydrogel" used herein refers to a network or scaffold of mixture of materials, molecules, polymers or substances that are combined by chemical bonds, including covalent, ionic and supramolecular bonds, or by any combination thereof, to form water-swellable but water-insoluble structures with a polymerized solid, semi-solid or semi-liquid texture, which is used as tissue-engineering scaffold. In an embodiment, the hydrogel is polymerized. By "polymerized" is referred herein as to a state of solid or semi-solid hydrogel that is a water-swollen, and cross-linked polymeric network produced by the simple reaction of one or more monomers.

The preparation of a hydrogel according to step a) preferably further comprises the steps of:
a-i) preparing a polymer solution comprising at least one polysaccharide, preferably agarose, wherein the polymer solution is maintained in a liquid state,
a-ii) adding to the polymer solution of i), in any order, fibrin or a source of fibrin, a source of clotting factor of type II, a source of calcium, an antifibrinolytic agent, optionally a cell population comprising stromal cells, optionally other clotting factors, and
a-iii) allowing the resulting solution of step ii) to polymerize, preferably at about 37°C-20°C, thereby forming a hydrogel.

By "maintained in a liquid sate" is referred herein as maintained at a temperature that is high enough to avoid spontaneous polymerization of the polymer solution. Said temperature may be between 37-45 °C, preferably about 40°C. Therefore, the resulting product of step a-iii) is the hydrogel of step a).

In an embodiment, the hydrogel of step a) is a fibrin matrix, i.e., a polymerized fibrin matrix. Fibrin matrices are very versatile, so as employed for the preparation of various artificial tissues. For this reason, polymer solution of step a-i) comprises at least one polysaccharide and fibrin. Said polysaccharide is employed to provide strength and consistency to the tissue and is suitably soluble therein. Examples of polysaccharides which may be employed in step a-i) of the method of the present invention are, but not limited to, agar, agarose, alginate, chitosan or carrageenan, or any combination thereof. Preferably, the at least one polysaccharide is agarose. Preferably, the hydrogel comprises agarose and fibrin.

Agarose is a polysaccharide consisting of galactose alpha and beta extracted from algae of *Gracillaria* and *Gellidium* genres. Agarose, other polysaccharides against which may be employed in a-i) of the present invention, has the advantage of forming an inert matrix from immunologically. There are different types of agarose vary in their physical and chemical properties such as, for example, the gelation temperature, gel strength and / or porosity. Preferably, the agarose is an agarose with low melting point, ie, one agarose which repolymerise and solidify at a temperature, preferably less than 65 °C and, more preferably, less than 40 °C; in this way it can be employed to prepare tissue to very low temperatures, minimizing the likelihood of cell death. In a more preferred embodiment, the agarose employed is of type VII. In a further preferred embodiment, the agarose comprised in the hydrogel of step a), preferably agarose type VII, is at a final concentration between 0.2 and 6 g/L, preferably between 0, 3 and 3 /L, and more preferably between 0.5 and 2 g/L. However, lower or higher concentrations could also be used.

In another preferred embodiment of the first aspect of the invention or of any of its embodiments, the final concentration of agarose in the hydrogel of step a), is between 0.01% and 5% (w/v), preferably between 0.05% and 3% (w/v), between 0.05% and 1% (w/v), between 0.05% and 0.6% (w/v), preferably 0.1%. The terms "% by weight" or "% wt" or "%w/v" are considered equivalents and can be used interchangeably herein and refer to the weight percentage relative to the total weight or volume of the solution or dispersion, unless otherwise specified. It is further noted that the hydrogel of the present invention can optionally further comprise proteoglycans.

The polymer solution of step a-i), which is in a liquid state, of the present invention can be easily prepared by dissolving the at least one polysaccharide, preferably agarose powder, in a suitable medium such as a buffer such as PBS. The solution is then heated to temperatures above 60°C, preferably to the boiling point, or preferably until a homogeneous solution is formed. The solution can be then maintained in a solid state at temperatures of about 4°C, and be pre-heated at about 80 to 100°C before use, in order to return it to its liquid state. In an embodiment, the polymer solution is allowed to cool down until it reaches between 45°C-40°C before proceeding with step a-ii) in orderto maintain it in a liquid state. In a further embodiment, step a-ii) is performed while the polymer solution is maintained in a liquid state, preferably at an average temperature of between 45°C-40°C, to avoid premature polymerization.

As stated above, fibrin or a source of fibrin is added in step a-ii). Preferably, the source of fibrin is fibrinogen which will be converted into fibrin in the presence of a source of calcium, as explained below. In a preferred embodiment, the final concentration of fibrin in the hydrogel of step a) is between 1 and 10 g / L. In a more preferred embodiment, the final concentration of fibrin in the hydrogel of step a) is between 2 and 4 g / L. However, lower or higher concentrations could also be used. In a preferred embodiment, the source of fibrin is fibrinogen, and the fibrinogen is autologous. However, the fibrin or the fibrinogen may also be allogenic or xenogenic origin. In addition to fibrin, the resulting solution of step a-ii) may contain other coagulation factors, such as factor XIII.

Other components added in step a-ii) are a source of clotting factor of type II and, optionally, a source of other clotting factors. The term "clotting factor", as used herein, refers to a component, usually a protein, present in blood plasma and intervening in the chain reaction that enables coagulation. They are thirteen clotting factors, designated by Roman numbers: I: fibrinogen or fibrin; II: thrombin or prothrombin; III: tissue factor or thromboplastin; IV: calcium; V: proaccelerin; VI: inactive or zymogen factor; VII: proconvertin; VIII: A antihemophilic factor or von Willebrand factor; IX antihemophilic factor B or Christmas factor; X: Stuart-Prower factor; XI: antihemophilic factor C; XII: Hageman Factor; XIII: fibrin stabilizing factor; XIV: Fitzgerald; XV: Fletcher; XVI: platelets; and XVII: Somocurcio. Preferably, other clotting factors are added in step a-ii) of the method of the present invention such as factor XIII.

In a preferred embodiment, the source of clotting factor of type II added in step a-ii) is thrombin or prothrombin. In a preferred embodiment, the thrombin or prothrombin is autologous. However, the thrombin or prothrombin may also be allogenic or xenogenic origin.

It is noted that the source of clotting factor of type II, the source of other clotting factors, and the fibrin or the source of fibrin may be the same source, which may be blood, preferably blood plasma. Preferably, the plasma is human plasma. Therefore, the embodiments of the step a-ii) of adding a source of clotting factor of type II, a source of other clotting factors, and fibrin or a source of fibrin can be substituted by a step of adding blood, preferably blood plasma, preferably human plasma. In this case, the amount of blood plasma in the hydrogel of step a) is of between 10-95%, preferably 30% to 95%, preferably 40% to 90%, most preferably 45% and 90% (v/v). The resulting solution of step a-ii) can also be prepared using a plasma derivative, such as, but not limited cryoprecipitate or fibrinogen concentrate.

In step a-ii), the solution comprising a source of fibrin is polymerized. As stated above, the source of fibrin may be preferably fibrinogen. Therefore, a fibrin matrix between the at least one polysaccharide, preferably agarose, and the components added in step ii) is formed. The formation of a fibrin matrix may be induced by polymerization of fibrinogen by thrombin induced in the presence of calcium. Fibrinogen is a high molecular weight protein that is present in blood plasma. Thrombin is a proteolytic enzyme which causes breakdown of the fibrinogen molecule in low molecular weight polypeptides and in fibrin monomers. Such monomers polymerized into dimers and subsequently joined together by covalent bonds, by factor XIII, activated by thrombin previously, and in the presence of calcium ions. However, the fibrin polymer may be degraded by a process called fibrinolysis. During fibrinolysis, plasminogen is converted to the active enzyme plasmin, by tissue plasminogen activator; plasmin binds to fibrin surface through its binding sites to produce polymer degradation fibrin.

The concentration of calcium salt should be sufficient to induce polymerization of fibrinogen. In a more preferred embodiment of this first aspect of the invention, the calcium source added in step a-ii) is a calcium salt such as, but not limited to, calcium chloride, calcium gluconate or a combination of both. In a more preferred embodiment, the calcium salt is calcium chloride, preferably a solution of calcium chloride at a final concentration of between 0.01% and 2% (w/v), preferably 0.1% or 0.2% (w/v) of CaCl₂. In a further preferred embodiment, the final concentration of calcium in the hydrogel of step a) is between 0.5 and 3 g/L. However, lower or higher concentrations could also be used.

To avoid fibrinolysis of the fibrin matrix, an antifibrinolytic agent is also added in step a-ii), wherein the antifibrinolytic agent may be, but not limited, epsilon aminocaproic acid, tranexamic acid or aprotinin. Preferably, the antifibrinolytic agent added in step ii) is tranexamic acid. Tranexamic acid is a synthetic product derived from amino acid lysine with high affinity binding sites plasminogen lysine; blocking these sites prevent plasminogen binding to the surface of activated fibrin, exerting an antifibrinolytic effect. Tranexamic acid has the advantage over other antifibrinolytic agents of animal origin, not transmitted diseases. Therefore, in a preferred embodiment, the antifibrinolytic agent is tranexamic acid. In a further preferred embodiment, the final concentration of antifibrinolytic agent, preferably tranexamic acid, in the hydrogel of step a) is between 1 and 2 g/L. However, lower or higher concentrations could also be used. Preferably, the final concentration of antifibrinolytic agent, preferably tranexamic acid, in the polymerized hydrogel of step a) is between 0.1% and 6%, preferably 0.5 and 3%, preferably 1.5% (w/v).

In order to better reproduce the dermal layer of the skin, a cell population comprising stromal cells may be added in step a-ii). The population comprising stromal cells can be autologous or xenogenic. By "stromal cells" is referred herein as connective tissue cells of any organ, preferably from the skin dermis.

Preferably, the stromal cells are fibroblasts or undifferentiated cells with the ability to differentiate into fibroblast cells. Preferably, the fibroblasts are human fibroblasts. The term "fibroblasts" used herein refers to is a resident cell type of the connective tissue. Fibroblast synthesize fibres and maintains the extracellular matrix of tissue in many animals. These cells provide a lattice-like structure (stroma) to many different tissues and play a crucial role in wound healing, being the most common cells of connective tissue. They are derived from primitive mesenchymal and pluripotent cells. In some embodiments, the population comprising stromal cell is a population comprising human fibroblasts, preferably human cancerous fibroblasts, human non-cancerous fibroblasts, or both. By "cancerous fibroblasts" is referred herein as malignant fibroblasts or fibroblasts residing within a tumor. Preferably, the population of fibroblasts comprises human non-cancerous (i.e., healthy) fibroblasts. Preferably, the fibroblasts are a primary culture of human fibroblasts. Fibroblasts can be obtained from any tissue or organ; however, it is preferred herein that the fibroblasts used are skin or dermal fibroblasts. Methods to isolate human primary fibroblasts can be found in, e.g., Garzon I, et al. Wharton's jelly stem cells: a novel cell source for oral mucosa and skin epithelia regeneration. Stem cells translational medicine. 2013;2(8):625-32; Carriel V, et al. Epithelial and stromal developmental patterns in a novel substitute of the human skin generated with fibrin-agarose biomaterials. Cells, tissues, organs. 2012;196(1):1-12.

In a preferred embodiment of the first aspect of the invention, the skin model may further comprise at least one of the components selected from a list consisting of alginate, gelatin from several origins (fish, bovine, porcine skin), fibrin or a source of fibrin from several origins (bovine, porcine, human plasma), intraarticular sodium hyaluronate, silk fibroin and sericine, xanthan, genipin, agar, chitosan, nanocellulose, proteoglicans (dermatan sulphate, hyaluronic acid, keratin, chondroitin sulphate, keratan sulphate), elastin, and other collagen types such as collagen I, II, III y IV, or their methacryloyl variants, or any combination thereof. These components, if added, are preferably be added in step a-ii).

Therefore, the resulting product of step ii) comprises or consists of a liquid polymer or polymer solution comprising at least one polysaccharide, preferably agarose, fibrin or a source of fibrin, a source of clotting factor if type II, preferably thrombin or prothrombin, a source of calcium, preferably calcium chloride, and an antifibrinolytic agent, preferably tranexamic acid. Optionally, the liquid polymer or polymer solution further comprises other clotting factors and a cell population comprising stromal cells.

Step a-iii) of the method of the first aspect or any of its embodiments comprises or consists of allowing said resulting product of step ii) to polymerize. By "allowing the resulting solution of step a-ii) to polymerize" is referred herein as creating the suitable conditions to allow the product of step a-ii) (i.e., the liquid polymer) to form chemical crosslinks. Once step a-iii) is performed, the resulting product is the hydrogel of step a). Cross linking is a stabilization process in polymer chemistry which leads to multidimensional extension of polymeric chain resulting in network structure. Cross-link is a bond which links one polymer chain to other. It can be ionic or covalent. Cross linking changes a liquid polymer into 'solid' or 'gel' by restricting the ability of movement. When polymer chains are linked together by cross-links, they lose some of their ability to move as individual polymer chains. A liquid polymer (where the chains are freely flowing) can be turned into a 'solid' or 'gel' by cross-linking the chains together. Cross linking increases the molecular mass of a polymer. Cross-linked polymers are important because they are mechanically strong and resistant to heat, wear and attack by solvents. The formation of said crosslinks may happen naturally when temperatures of below 40°C, preferably 37°C, are reached. Thus, the polymer solution of step a-i) may be prepared by heating the agarose in a suitable buffer, such as PBS, until a homogeneous solution is prepared, and letting it cool down to temperatures below 40°C, preferably 37°C, so that the polymer solution jellifies, thereby forming the hydrogel of step a).

Since the cell population comprising stromal cells, preferably fibroblasts, may be added in step a-ii), once the polymerization of the polymer solution is carried out in step a-iii), said cell population will be embedded in the resulting polymerized hydrogel of step a).

It is noted that step a-ii) and step a-iii) can be carried out in any order, or even simultaneously. In some embodiments, if no cells are added in step a-ii) before proceeding to step b) the hydrogel is sterilized.

Steps b) adding to the hydrogel of step a), preferably on its surface, a cell population comprising squamous carcinoma keratinocyte cells.

In another preferred embodiment of the first aspect of the invention or of any of its embodiments, the population of squamous carcinoma keratinocytes are squamous carcinoma epidermal keratinocytes, preferably from human origin.

In an embodiment, the squamous carcinoma epidermal keratinocytes, preferably human squamous carcinoma epidermal keratinocytes, are primary cells or cells directly derived from a patient or taken directly from living tissue. Thus, the population comprising squamous carcinoma keratinocytes may be autologous. The possibility that all artificial tissue components are autologous allows such tissue to be used without immunosuppression of the subject transplanted. However, components of artificial tissue can also be allogenic origin, i.e., may come from a different person to the one who is going to receive the artificial tissue, in which case it is said that its origin is xenogenic. This opens the possibility that the artificial tissue is prepared in advance when needed urgently, although in this case it would be advisable to proceed to immunosuppression of the subject to which the artificial tissue is transplanted. Thus, in some embodiments, the population comprising squamous carcinoma keratinocytes are allogenic or have a xenogenic origin.

In another embodiment, the population comprising squamous carcinoma keratinocytes is a population comprising squamous carcinoma keratinocytes derived from an established line that has not been directly or freshly isolated from a patient. Preferably, the squamous carcinoma epidermal keratinocytes are A431 cell line, which is a human cell line derived from an 85-year-old female suffering from Epidermoid Carcinoma that were established from solid tumors by Giard DJ, et al. In vitro cultivation of human tumors: establishment of cell lines derived from a series of solid tumors. J. Natl. Cancer Inst. 51: 1417-1423, 1973. Preferably, the A431 are A431 ATCC CRL-1555^{™}.

In some other embodiments, the population of squamous carcinoma epidermal keratinocytes is a heterogeneous population comprising other epithelial cells, preferably cancerous and/or non-cancerous epithelial cells. In some embodiments, the cell population comprising squamous carcinoma keratinocyte cells further comprises embryonic stem cells (ESCs), non-embryonic or adult stem cells, progenitor cells, and/or iPS cells.

In some embodiments, the population of squamous carcinoma keratinocytes further comprises non-cancerous keratinocytes. By "non-cancerous keratinocytes" is referred herein as healthy keratinocytes. Preferably, the keratinocytes (both cancerous and non-cancerous) are patient-derived keratinocytes.

All cells described herein (steps b) and c) of the method of the first aspect or any of its embodiments) can be obtained by different methods described in the art and may depend on the particular cell type in question. Some of these methods are, for example, but not limited to biopsy, mechanical processing, enzymatic treatment (eg, without limitation, trypsin or collagenase type I), centrifugation, erythrocyte lysis, filtration, culture media or media that favoring selective proliferation of the cell type or immunocytometry.

Preferably, the cells added in step b) are added on the surface of the hydrogel of step a).

Optional step c) of adding to the hydrogel prior to, simultaneously to, or subsequently to step b), preferably on its surface, a cell population comprising non-cancerous keratinocyte cells, and/or a cell population comprising mesenchymal cells, and

Optionally, apart from the cells of step b), other type of cells can be added to the hydrogel of step a). Please note that this step is optional and can be performed simultaneously, before, or after, step b).

In one embodiment, a cell population comprising keratinocyte cells is added to the hydrogel of step a) or to the product of step b). In one embodiment, a cell population comprising mesenchymal cells is added to the hydrogel of step a) or to the product of step b). In a further embodiment, both cell populations (comprising keratinocytes cells and comprising mesenchymal cells) are added to the hydrogel of step a) or to the product of step b). It is noted that, in addition or alternatively, undifferentiated cells, preferably epithelial cells, with the ability to differentiate into such cells (keratinocytes cells and/or mesenchymal cells) can also be added, such as adult stem cells.

The population comprising keratinocytes can be autologous or xenogenic. Preferably, the keratinocytes are human keratinocytes. In some embodiments, the population comprising keratinocytes is a population comprising human cancerous keratinocytes, human non-cancerous keratinocytes, or both. Preferably, the keratinocytes are non-cancerous keratinocytes, or undifferentiated cells with the ability to differentiate into non-cancerous keratinocytes. By "non-cancerous keratinocytes" is referred herein as healthy keratinocytes. Preferably, the non-cancerous keratinocytes are human non-cancerous keratinocytes. Preferably, the non-cancerous keratinocytes are a primary culture of human non-cancerous keratinocytes. Keratinocytes can be obtained from any tissue or organ; however, it is preferred herein that the keratinocytes used are skin keratinocytes.

The population comprising mesenchymal cells can be autologous or xenogenic. Preferably, the mesenchymal cells are human mesenchymal cells. Preferably, the human mesenchymal cells are able to differentiate into keratinocytes. Preferably, the mesenchymal cells are non-cancerous mesenchymal cells, or undifferentiated cells with the ability to differentiate into non-cancerous mesenchymal cells. By "non-cancerous mesenchymal cells" is referred herein as healthy mesenchymal cells. Preferably, the non-cancerous mesenchymal cells are human non-cancerous mesenchymal cells. Preferably, the non-cancerous mesenchymal cells are a primary culture of human mesenchymal cells, preferably a primary culture of human non-cancerous mesenchymal cells. Mesenchymal cells can be obtained from any tissue or organ; however, it is preferred herein that the mesenchymal cells used are skin derived mesenchymal cells.

Preferably, the cells added in step c) are added on the surface of the hydrogel.

Importantly, the number of cells added, and their proportions, will define behaviour of the biomimetic squamous cell carcinoma skin model. Preferably, the total number of cells added to the hydrogel (i.e., the cells of step b) alone or the sum of steps b) and c)) is at least 1×10³ cells per ml of hydrogel. Preferably, the total number of cells added is at least 1×10⁴, 1×10⁵, 1×10⁶, 1×10⁷, or more than 1×10⁸, cells per ml of hydrogel. Preferably, the total number of cells is of about 1000 to 1 million of cells per ml of hydrogel, preferably between 10.000 to 1 million, or 100.000 to 1 million. Regarding the proportions between the different cell populations in if step c) is performed, it is preferred that a higher number of cells of step c) than of cells b) are added. In a preferred embodiment, about 3, 4, preferably 5 times more of cells from c), preferably fibroblasts, than cells from b) are added.

In the context of the present invention, it is to be understood that any cell populations described herein is preferably a homogeneous or substantially pure population or a mixed cell population enriched in the specific cell population (stromal cell, keratinocytes, mesenchymal). By "homogeneous" or "substantially pure population" is referred as to at least about 75%, at least about 80%, at least about 85%, at least about 90%, at least about 95%, at least about 98%, at least about 99% or 100% of the cells in the composition are the cells defined herein (stromal, keratinocytes, mesenchymal). The cell populations are added in a suitable medium forming a cell suspension including pharmaceutically acceptable liquid medium.

Step d) culturing the cells of the product resulting from (b) or, optionally, the product resulting from (c) in a suitable culture medium to allow the cells to grow and expand and/or to differentiate to provide a biomimetic squamous cell carcinoma skin model.

Once the steps a) to b) or, optionally, a) to c), are performed, the product resulting from said steps is allowed to stand on a support to form the hydrogel matrix comprising fibrin and at least one polysaccharide, preferably agarose. Supports which can be used are, for example, but not limited to tissue culture plates or porous cell culture inserts. Preferably, such supports will be sterile prior use.

Step d) comprises culturing the cells in or on the resulting product of step a), so that they are able to grow and proliferate to provide a biomimetic squamous cell carcinoma skin model. Preferably, the cells of step d) proliferate on the surface and/or embedded in the resulting product of step a). The cells during step d) are allowed to proliferate until they reach an adequate number until they reach typically at least 70% confluency, advantageously at least 80% confluency, preferably at least 90% confluence, more preferably at least 95% confluency and, even more preferably at least 100% confluency. During the time the cells are maintained in a suitable culture medium, the culture medium in which are can be partially or completely replaced by new medium replacement ingredients depleted and eliminate metabolites and catabolites potentially harmful.

In an embodiment, if mesenchymal cells are added, step d) comprises a further step of differentiating said mesenchymal cells into mature keratinocytes. Media and conditions for proper differentiation are known by the skilled person.

A skill person would know how to prepare a suitable medium and under standard conditions. For example, cells such as fibroblasts can be cultured in commercial DMEM medium supplemented with 1% antibiotic solution and 10% fetal bovine serum (DMEMc). The same medium can also be used to grow the A431 cell line. Ker-CT cell lines can be grown in a 3:1 mixture of DMEM and Ham F12 culture medium supplemented with 10% foetal bovine serum (FBS), 1% antibiotics, 24 g/ml adenine, 0.4 g hydrocortisone/ml, insulin 5 g/ml, epidermal growth factor 10 ng/ml, triiodothyronine 1.3 ng/ml, and cholera toxin 8 ng/ml (all from Sigma-Aldrich). Standard conditions for cell culture are considered 37°C and 5% CO₂.

In an embodiment, the epithelial surface (i.e., keratinocytes) is exposed to air to promote proper stratification and epithelial maturation of the cells, maintaining the matrix comprising the cells of step d) submerged in medium culture (art liquid air). Therefore, in a preferred embodiment, the method of the invention, comprises a step in which the resulting product of step a) to b) or, optionally, a) to c), is exposed, at least partially, to air.

The time of culturing of step d) is not limiting and it depends on the desired maturity degree of the skin model. If a skin model imitating early stages of tumour development is desired, the time of culturing of step d) is preferably short, i.e., between 1-7 days of culture. However, if a skin model imitating late-stage tumour development, up to 14 days, or more than 15 days, of culture are recommended. Preferably, step d) comprises a first step in which the resulting product of step a) to b) or, optionally, a) to c), is completely submerged in a suitable medium for at least 1, preferably 2, 3, 4, 5, 6, 7, 8, 9, or 10 days; followed by a second step in which the resulting product of the first step is cultured in an air-liquid interface system for at least 1, 2, 3, 4, 5, 6, 7, 8, 9, 10, 11, 12, 13, preferably 14 days. Preferably, step d) comprises a first step of completely submerging the resulting product of step a) to b) or, optionally, a) to c), in a suitable medium for at least 1-5 days, preferably 2 days, followed by a second step of culturing the resulting product of the first step in an air-liquid interface system for at least 12-16 days, preferably 10, more preferably 14 days.

It is noted that each embodiment disclosed under steps a) to d) is contemplated as being applicable to each of the other disclosed embodiments. Thus, all combinations of the various elements described herein are within the scope of the invention.

Preferred embodiments of the first aspect are:
In an embodiment, the method for the *in vitro* production of a biomimetic squamous cell carcinoma skin model, the method comprising the steps of:
a) providing a hydrogel comprising agarose and fibrin wherein the hydrogel is prepared following the steps of:
   i. preparing a polymer solution comprising agarose, wherein the polymer solution is maintained in a liquid state,
   ii. adding to the polymer solution of i), in any order, an antifibrinolytic agent, a source of calcium, fibrin or a source of fibrin, a source of clotting factor of type II, and optionally a cell population comprising stromal cells, and
   iii. allowing the resulting solution of step ii) to polymerize, preferably at 37°C,
b) adding to the hydrogel of step a), preferably on its surface, a cell population comprising squamous carcinoma keratinocyte cells,
c) optionally, prior to, simultaneously to, or subsequently to step b), adding to the hydrogel, preferably on its surface, a cell population comprising non-cancerous keratinocyte cells, and/or a cell population comprising mesenchymal cells, and
d) culturing the cells of the product resulting from b) or, optionally, the product resulting from c), in a suitable culture medium to allow the cells to grow and expand and/or to differentiate to provide a biomimetic squamous cell carcinoma skin model.

In another embodiment, the method for the *in vitro* production of a biomimetic squamous cell carcinoma skin model, the method comprising the steps of:
a) providing a hydrogel comprising agarose and fibrin wherein the hydrogel is prepared following the steps of:
   i. preparing a polymer solution comprising agarose, wherein the polymer solution is maintained in a liquid state,
   ii. adding to the polymer solution of i), in any order, an antifibrinolytic agent, a source of calcium, blood plasma, and optionally a cell population comprising stromal cells, and
   iii. allowing the resulting solution of step ii) to polymerize, preferably at 37°C,
b) adding to the hydrogel of step a), preferably on its surface, a cell population comprising squamous carcinoma keratinocyte cells,
c) optionally, prior to, simultaneously to, or subsequently to step b), adding to the hydrogel, preferably on its surface, a cell population comprising non-cancerous keratinocyte cells, and/or a cell population comprising mesenchymal cells, and
d) culturing the cells of the product resulting from b) or, optionally, the product resulting from c), in a suitable culture medium to allow the cells to grow and expand and/or to differentiate to provide a biomimetic squamous cell carcinoma skin model.

In another embodiment, the method for the *in vitro* production of a biomimetic squamous cell carcinoma skin model, the method comprising the steps of:
a) providing a hydrogel comprising agarose and fibrin, wherein the hydrogel is prepared following the steps of:
   i. preparing a polymer solution comprising agarose, wherein the polymer solution is maintained in a liquid state,
   ii. adding to the polymer solution of i), in any order, an antifibrinolytic agent, a source of calcium, human plasma, and a cell population comprising stromal cells, preferably fibroblasts, and
   iii. allowing the resulting solution of step ii) to polymerize, preferably at 37°C,
b) adding to the hydrogel of step a), preferably on its surface, a cell population comprising squamous carcinoma keratinocyte cells,
c) optionally, prior to, simultaneously to, or subsequently to step b), adding to the hydrogel, preferably on its surface, a cell population comprising non-cancerous keratinocyte cells, and/or a cell population comprising mesenchymal cells, and
d) culturing the cells of the product resulting from b) or, optionally, the product resulting from c), in a suitable culture medium to allow the cells to grow and expand and/or to differentiate to provide a biomimetic squamous cell carcinoma skin model.

In another embodiment, the method for the *in vitro* production of a biomimetic squamous cell carcinoma skin model, the method comprising the steps of:
a) providing a hydrogel comprising agarose and fibrin, wherein the hydrogel is prepared following the steps of:
   i. preparing a polymer solution comprising agarose, wherein the polymer solution is maintained in a liquid state, and wherein the agarose is at a final concentration of between 0.05% and 0.6% (w/v), preferably 0.1% (w/v);
   ii. adding to the polymer solution of i), in any order, an antifibrinolytic agent at a final concentration of 0.5 and 3%, preferably 1.5% (w/v), a source of calcium at a final concentration of between 0.01% and 2% (w/v), human plasma at a final concentration of between 10% and 95% (v/v), and a cell population comprising stromal cells, preferably fibroblasts, and
   iii. allowing the resulting solution of step ii) to polymerize, preferably at 37°C,
b) adding to the hydrogel of step a), preferably on its surface, a cell population comprising squamous carcinoma keratinocyte cells,
c) optionally, prior to, simultaneously to, or subsequently to step b), adding to the hydrogel, preferably on its surface, a cell population comprising non-cancerous keratinocyte cells, and/or a cell population comprising mesenchymal cells, and
d) culturing the cells of the product resulting from b) or, optionally, the product resulting from c), in a suitable culture medium to allow the cells to grow and expand and/or to differentiate to provide a biomimetic squamous cell carcinoma skin model.

In another embodiment, the method for the *in vitro* production of a biomimetic squamous cell carcinoma skin model, the method comprising the steps of:
a) providing a hydrogel comprising agarose and fibrin, wherein the hydrogel is prepared following the steps of:
   i. preparing a polymer solution comprising agarose, wherein the polymer solution is maintained in a liquid state, and wherein the agarose is at a final concentration of between 0.05% and 0.6% (w/v), preferably 0.1% (w/v);
   ii. adding to the polymer solution of i), in any order, tranexamic acid at a final concentration of 0.5% and 3%, preferably 1.5% (w/v), CaCl₂ at a final concentration of between 0.01% and 2% (w/v), human plasma at a final concentration of between 10% and 95% (v/v), and a cell population comprising fibroblasts, and
   iii. allowing the resulting solution of step ii) to polymerize, preferably at 37°C,
b) adding to the hydrogel of step a), preferably on its surface, a cell population comprising squamous carcinoma keratinocyte cells, preferably A431 cells,
c) optionally, prior to, simultaneously to, or subsequently to step b), adding to the hydrogel, preferably on its surface, a cell population comprising non-cancerous keratinocyte cells, and/or a cell population comprising mesenchymal cells, and
d) culturing the cells of the product resulting from b) or, optionally, the product resulting from c), in a suitable culture medium to allow the cells to grow and expand and/or to differentiate to provide a biomimetic squamous cell carcinoma skin model.

In another embodiment, the method for the *in vitro* production of a biomimetic squamous cell carcinoma skin model, the method comprising the steps of:
a) providing a hydrogel comprising agarose and fibrin, wherein the hydrogel is prepared following the steps of:
   i. preparing a polymer solution comprising agarose, wherein the polymer solution is maintained in a liquid state, and wherein the agarose is at a final concentration of between 0.05% and 0.6% (w/v), preferably 0.1% (w/v);
   ii. adding to the polymer solution of i), in any order, tranexamic acid at a final concentration of 0.5% and 3%, preferably 1.5% (w/v), CaCl₂ at a final concentration of between 0.01% and 2% (w/v), human plasma at a final concentration of between 10% and 95% (v/v), and a cell population comprising fibroblasts, and
   iii. allowing the resulting solution of step ii) to polymerize, preferably at 37°C,
b) adding to the hydrogel of step a), preferably on its surface, a cell population comprising squamous carcinoma keratinocyte cells, preferably A431 cells,
c) prior to, simultaneously to, or subsequently to step b), adding to the hydrogel, preferably on its surface, a cell population comprising non-cancerous keratinocyte cells, and
d) culturing the cells of the product resulting from b) or, optionally, the product resulting from c), in a suitable culture medium to allow the cells to grow and expand to provide a biomimetic squamous cell carcinoma skin model.

In another embodiment, the method for the *in vitro* production of a biomimetic squamous cell carcinoma skin model, the method comprising the steps of:
a) providing a hydrogel comprising agarose and fibrin, wherein the hydrogel is prepared following the steps of:
   i. preparing a polymer solution comprising agarose, wherein the polymer solution is maintained in a liquid state, and wherein the agarose is at a final concentration of between 0.05% and 0.6% (w/v), preferably 0.1% (w/v);
   ii. adding to the polymer solution of i), in any order, tranexamic acid at a final concentration of 0.5% and 3%, preferably 1.5% (w/v), CaCl₂ at a final concentration of between 0.01% and 2% (w/v), human plasma at a final concentration of between 10% and 95% (v/v), and a cell population comprising fibroblasts, and
   iii. allowing the resulting solution of step ii) to polymerize, preferably at 37°C,
b) adding to the hydrogel of step a), preferably on its surface, a cell population comprising squamous carcinoma keratinocyte cells, preferably A431 cells,
c) prior to, simultaneously to, or subsequently to step b), adding to the hydrogel, preferably on its surface, a cell population comprising a primary culture of non-cancerous human keratinocytes, and
d) culturing the cells of the product resulting from b) or, optionally, the product resulting from c), in a suitable culture medium to allow the cells to grow and expand to provide a biomimetic squamous cell carcinoma skin model.

In a **second aspect,** the present invention relates to a non-melanoma skin cancer model, preferably a squamous cell carcinoma skin model, obtained or obtainable by the method of the first aspect of the invention or by any of its embodiments, hereinafter "squamous cell carcinoma skin model of the invention". In a **third aspect,** the present invention provides a composition comprising the squamous cell carcinoma skin model of the invention. So, in another aspect, the present invention relates to a composition, hereinafter "composition of the invention", comprising the squamous cell carcinoma skin model of the invention as obtained or obtainable by the method of the first aspect of the invention or by any of its embodiments.

Lyophilization of the squamous cell carcinoma skin model obtained according to any of the above two aspects may be carried out or done in order to obtain a stable preparation of the same. The term "lyophilizing" or "lyophilization" as used herein refers to the rapid freezing and dehydration of the frozen product under high vacuum and is also commonly referred to as "freeze-drying".

The squamous cell carcinoma skin model obtained according to any of the above two aspects including its composition or lyophilized form finds several uses and applications. Accordingly, a **fourth aspect** of the present invention provides the in vitro use of the non-melanoma skin cancer model, preferably the squamous cell carcinoma skin model, as defined in the second aspect or the composition of the invention, for the pharmacological development and preclinical screening of chemotherapeutic drugs, and basic study of the fundamental biology of cancer and the processes that mediate tumor proliferation, growth, metastasis and invasion.

The squamous cell carcinoma skin model or the composition of the invention can be used as *in vitro* disease model of skin pathologies, injuries, or tumor. In addition, the 3D-bioprinted Malignant Model of the present invention or the composition of the invention can be comprised in an implant to form a tissue scaffold that can be implanted into a study subject, such as mice, for use in pharmacological development and preclinical screening of chemotherapeutic drugs, and basic study of the fundamental biology of cancer and the processes that mediate tumor proliferation, growth, metastasis and invasion.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Additional objects, advantages and features of the invention will become apparent to those skilled in the art upon examination of the description or may be learned by practice of the invention.

The following examples and drawings are provided by way of illustration and are not intended to be limiting of the present invention.

### EXAMPLES

### EXAMPLE 1: Biofabrication of the non-melanoma skin cancer biomimetic model

### Sampling and primary cell cultures obtention

In the present invention several cell sources have been used to generate the NMC model. Specifically for the constitution of the epithelium, two cell lines widely described in the literature were used: the A431 cell line and the Ker-CT cell line, both acquired in ATCC. The A431 cell line was established from an epidermoid carcinoma of an 85-year-old woman derived from solid tumors (128). The Ker- CT cell line is an immortalized cell line derived from normal human epidermal keratinocytes from a healthy male patient; this immortalization was carried out by overexpression of telomerase and Cdk4 using retroviral vectors (129) and was shown to maintain the typical characteristics of human keratinocytes in organotypic cultures (130, 131).

Normal human fibroblasts were used for the dermal stroma generation which were isolated from healthy tissue biopsies as previously described (6, 7, 10). Briefly, fibroblasts were obtained from small biopsies of normal human skin from healthy donors undergoing minor surgery at the Department of Plastic Surgery of the Hospital Universitario Virgen de las Nieves in Granada, Spain. The connective tissue was first mechanically disintegrated into small fragments and then enzymatically digested using a mixture of Dulbecco's modified Eagle's modified Eagle's (DMEM) and 2 mg/ml Clostridium histolyticum collagenase I (Gibco BRL Life Technologies, Karlsruhe, Germany) at 37°C. The detached fibroblasts were collected by centrifugation and expanded in culture flasks (7, 8, 44).

Fibroblasts were cultured in commercial DMEM medium supplemented with 1% antibiotic solution and 10% fetal bovine serum (DMEMc). The same medium was also used to culture the A431 cell line. The Ker-CT cell line was cultured in a mixture 3: 1 of DMEM and Ham F12 culture medium supplemented with 10% fetal bovine serum (FBS), 1% antibiotics, 24 g/ml adenine, 0.4 g/ml hydrocortisone, 5 g/ml insulin, 10 ng/ ml epidermal growth factor, 1.3 ng/ml triiodothyronine, and 8 ng/ ml cholera toxin (all from Sigma-Aldrich) (7, 58, 65). We refer to this medium as QC medium.

Both cell lines were maintained under standard culture conditions (37°C and 5% CO₂), the culture medium was renewed every three days and confluence was monitored daily by phase contrast microscopy (Nikon, Eclipse Ti-U). Subconfluent cultures were treated with trypsin-EDTA (Sigma Aldrich) and subcultured into new culture flasks.

### Carcinoma and normal human skin hydrogels generation.

Carcinoma and normal human skin models were generated using 0.4 µm 1.1 cm² porous inserts (Starstedt, Germany), as previously described (7, 8, 59). Firstly, the stroma was generated using a mixture of human fibrin obtained from blood plasma and 0.1% type VII agarose in PBS. To produce the 1 ml dermal substitute, 760 µl of human plasma was added to 125 µl of DMEM. To prevent in-gel fibrinolysis, the mixture was supplemented with 15 µl tranexamic acid (Amchafibrin, Fides Ecopharma, Spain). Then 50 µl of 2% CaCl₂ was added to induce fibrin polymerization, followed by the immediate addition of 50 µl of melted concentrated agarose (2% in PBS) at 40°C. The final concentration of agarose in the stroma substitute was 0.1%. Finally, the mixture was rapidly aliquoted onto porous inserts and allowed to solidify at 37°C for 2 h.

Then, different cell sources will be incorporated into the hydrogel to configure the following study groups which could reproduce the different stages of the non-melanoma skin cancer extension. In addition, to study the influence of cell stroma in orthotypic skin models, cellular and acellular stroma will be generated. Accordingly, the following experimental groups were configured (Figure 1):
- Control group: orthotypic artificial human skin generated with a stroma containing 300,000 human skin fibroblasts and an epithelium composed of 56,500 non-pathologic human keratinocytes of the KerCT line.
- Acellular Control Group: artificial human skin with acellular stroma and an epithelium composed of 56,500 non-pathologic human keratinocytes of the KerCT line.
- SCC group: non-melanoma skin cancer at different histological stages of extension of malignant cells with orthotypic artificial stroma. This model was generated with a stroma containing 300,000 human skin fibroblasts and an epithelium composed of 56,500 pathological human SCC keratinocytes of the A431 cell line.
- Acellular SCC group: non-melanoma skin cancer at different histological stages of extension of malignant cells with artificial acellular stroma. This model was generated with an acellular stroma and epithelium composed of 56,500 pathological human SCC keratinocytes of the A431 cell line.

Of note, the fibroblasts were embedded in the stroma as they were added during the generation of the hydrogel, while keratinocytes were added to the surface of the hydrogel. Finally, skin models were cultured for 2 days submerged in culture medium and then subjected to an air-liquid culture technique for 14 days to induce proper differentiation of multilayered epithelium. Tissues were maintained in culture at 37°C with 5% CO₂. Samples were taken for analysis at the time of the air-liquid phase change and every 2 days thereafter to see the temporal evolution of the SCC. See figure 1.

### Histological and structural characterization

Histological, histochemical and immunohistochemical (IHC) techniques were used to determine the structural patterns in the different models generated in this invention. For histological analyses, skin models were fixed in 10% neutral formalin and embedded in formalin. Histological sections were subsequently stained with hematoxylin-eosin (HE). In each sample, integrity parameters, cell morphology and stromal structures were determined. For ECM analysis, alcian blue (AB), periodic acid of Schiff (PAS) and picrosirius red (PS) staining methods were used to study the presence of glycosaminoglycans, carbohydrates and collagen fibers. In order to determine the biological behavior of tumor cells versus non-tumor skin, IHC techniques were used. Cell proliferation, expression of basal and surface cytokeratins, intercellular junctions and basement membrane formation and/or degradation were evaluated. The antibodies used for IHC detection are summarized in Table 1.

| **Table 1: Antibodies used for IHC detection** | |
|---|---|
| **Antibody** | **Reference** |
| Rabbit anti-cytokeratin 5 | Master Diagnostica, Granada, Spain, cat. Nº. MAD-000491QD |
| Mouse anti-cytokeratin 10 | Master Diagnostica, Granada, Spain, cat. Nº. MAD-000150QD |
| Mouse anti-cytokeratin (AE1-AE3) | Master Diagnostica, Granada, Spain, cat. Nº. MAD-001000QD |
| Rabbit Anti-Human Ki-67 | (Clone SP6) Master Diagnostica, Granada, Spain, cat. Nº. MAD-000310QD |
| Mouse anti-human PCNA | (Clone PC10) Master Diagnostica, Granada, Spain, cat. Nº. MAD-000903QD |
| Mouse anti-claudin-1 | Master Diagnostica, Granada, Spain, cat. Nº. MAD-000523QD |
| ImmPRESS^{™} anti-rabbit IgG (peroxidase) | Vector^{®} Laboratories; MP-7401 |
| ImmPRESS^{™} anti-mouse IgG (peroxidase) | Vector^{®} Laboratories; MP-7402 |

The application of the IT techniques and protocols described in the methodological section allowed the generation of new models of human non-melanoma skin cancer based on fibrin-agarose biomaterials.

Histological analysis of the non-pathological artificial skin model used as a control revealed the presence of an epithelium on the fibrin-agarose dermal substitute (Figure 2), with the presence of a unicellular layer of keratinocytes at the first time of development in submerged culture. When the epithelium was exposed to direct contact with air, 2-3 layers of epithelial cells could be identified at time 2 (Figure 2) which would tend to adopt a squamous morphology. Finally, at end time, after 12 days of air-liquid culture, these cells differentiated into a mature epithelium in which the most superficial layer showed evident signs of desquamation and a stratified epidermis with 8-10 layers. This epithelial maturation was clearly favored by the presence of dermal fibroblasts in the stroma. In this case, it could be observed that, even in the immersed state, keratinocytes present a flat or pavement-like morphology reflecting increased cell differentiation. Likewise, the analysis of the artificial human dermis generated by bioengineering showed an adequate development of fibroblasts in the hydrogel with a progression in their proliferation according to the experimental time. It is noteworthy to observe how the arrangement of fibroblasts along the hydrogel is not homogeneous, and two differentiated zones were established. One closer to the epithelial barrier, with a higher cell density, and another located underlying it where the cell density decreases, which may reflect the creation of a laxer papillary layer and a denser and deeper reticular layer found in the configuration of the human dermis (Figure 2, B), although without the typical ridges and papillae of the native tissue.

In the SCC pathologic model, several peculiarities were found over the studied time. First, we found that in the early stages of disease prominent tumor features such as aggregation of tumor cells and rapid invasion of the acellular stroma were appreciated, which is controlled, in the case of the cellular stroma, by the presence of fibroblasts. Subsequently, late-stage disease models were generated in which the epithelium was totally occupied by SCC cells in which spatial disorganization predominates and where some typical tumor structures such as secretory granules are evident (Figure 3).

During the progression of non-pathological skin and ex *vivo* disease, keratinocytes and fibroblasts behave differently. In the normal skin model, synthesis of typical ECM components such as carbohydrates, proteoglycans or collagen occurs, which are revealed by PAS, AB or PS staining, respectively. In the non-pathological skin control group, high expression of these components was found after 14 days in culture, especially in the area of the dermis closest to the epithelium, an area of high deposition of collagen and proteoglycan components (Figure 3). The IHC study revealed a subtle expression of type IV collagen in the dermo-epidermal barrier in the non-pathologic model and especially in the model generated with a cellular dermis (Figure 5). In contrast, SCC cells do not differentiate toward normal phenotypes and instead tend to migrate rapidly to the dermis when they do not encounter cellular impediment there in the acellular dermis group. However, the positive reaction of these components in some secretory granules of the neoplastic epithelium is striking.

IHC is a key tool in the differential diagnosis between different types of cancer of similar appearance (140, 141). In this sense, in this work we performed an IHC study of typical epithelial and tumor markers that revealed a progressive expression of the typical biomarkers of CSCC in the pathologic group (Figure 4 and 5).

First, the analysis of epithelial markers of CKs expression showed high analogy between both models (control and carcinoma), except for CK10, which showed opposite trends (Figure 4). The expression of CK5, a marker of the basal strata of the skin, was found to be intense in all models, highlighting the inability to generate fully differentiated surrogates under the present culture conditions and temporality of study. However, CK10 was differentially expressed, and in the case of the non-pathologic skin model, it was found only at the apical level in the most superficial strata, highlighting the terminal cell differentiation of these keratinocytes. Such differentiation did not occur in the case of the pathologic model of SCC, since no positive reaction to this marker was found at any of the studied times. Finally, the study of the cell proliferation markers Ki67 and PCNA demonstrated a high proliferative capacity in the non-pathologic artificial skin and epidermoid skin carcinoma model (Figure 5). Moreover, interestingly, the epidermal layer of the pathologic model showed a typical cell contact of tumor aggregation, which was highlighted by the high expression of intercellular binding proteins such as claudin (Figure 5).

### Conclusions

1. The application of the Tissue Engineering protocols developed in the present invention allowed the successful generation of two human skin cellular squamous cell carcinoma models based on fibrin-agarose hydrogels: a human non-melanoma skin cancer model with acellular stroma and a human non-melanoma skin cancer model with a cellular stroma. Both models were shown to be highly biomimetic with respect to native human skin controls.
2. The histological analysis carried out on the non-melanoma skin cancer models developed shows their suitability to reproduce ex *vivo* the structure and pathophysiology of native human skin. Specifically, it was successfully generated a new model of non-melanoma skin cancer to study its development and progression in a controlled and reproducible system. This model showed characteristics of tumor dissemination, invasion of tumor cells into the stroma and spatial disorganization, all compatible with an orthotopic epidermoid carcinoma. The study of human skin differentiation and maturation markers showed that the model is able to reproduce the main components of the extracellular matrix of normal skin, especially in the non-melanoma skin cancer model with stromal cells, as well as the main biomarkers of the neoplastic epithelium, with poorly differentiated keratinocytes with high proliferation capacity. Consequently, the non-melanoma skin cancer models generated in the present invention could be useful for the study of the pathophysiology of this type of cancer, as well as for the ex *vivo* evaluation of antitumor agents and new therapeutic protocols.

## Claims

1. A method for the *in vitro* production of a biomimetic squamous cell carcinoma skin model, the method comprising the steps of:
a) providing a hydrogel comprising agarose and fibrin,
b) adding to the hydrogel of step a), preferably on its surface, a cell population comprising squamous carcinoma keratinocyte cells,
c) optionally, prior to, simultaneously to, or subsequently to step b), adding to the hydrogel, preferably on its surface, a cell population comprising non-cancerous keratinocyte cells, and/or a cell population comprising mesenchymal cells, and
d) culturing the cells of the product resulting from (b) or, optionally, the product resulting from (c), in a suitable culture medium to allow the cells to grow and expand to provide a biomimetic squamous cell carcinoma skin model.

2. The method according to claim 1, wherein the preparation of a hydrogel according to step a) comprises the steps of:
i. preparing a polymer solution comprising agarose, wherein the polymer solution is maintained in a liquid state,
ii. adding to the polymer solution of i), in any order, an antifibrinolytic agent, a source of calcium, fibrin or a source of fibrin, a source of clotting factor of type II and, optionally, a cell population comprising stromal cells, and
iii. allowing the resulting solution of step ii) to polymerize, preferably at 37°C.

3. The method according to claim 2, wherein the cell population comprising stromal cells is added in step ii), and wherein said stromal cells are preferably fibroblasts.

4. The method according to claims 2 or 3, wherein the agarose comprised in the polymer solution of step i) is at a final concentration of between 0.05 and 0.6% (w/v), preferably 0.1%.

5. The method according to any of claims 2 to 4, wherein the clotting factor of type II added in step ii) is thrombin or prothrombin.

6. The method according to any of claims 2 to 5, wherein the source of clotting factor of type II and wherein the source of fibrin added in step ii), is blood plasma, and wherein the blood plasma added is at a final concentration of between 10% and 95% (v/v).

7. The method according to any of claims 2 to 6, wherein the antifibrinolytic agent added in step ii) is at a final concentration of between 0.5% and 3%, preferably 1.5% (w/v).

8. The method according to any of claims 2 to 7, wherein the antifibrinolytic agent added in step ii) is tranexamic acid.

9. The method according to any of claims 2 to 8, wherein the source of calcium added in step ii) is CaCl₂.

10. The method according to any of claims 1 to 9, wherein the population comprising non-cancerous keratinocytes cells of step c) are added and is preferably a primary culture of non-cancerous human keratinocytes.

11. The method according to any of claims 1 to 10, wherein the total number of cells added to the hydrogel is of about 1000 to 1 million of cells per ml of hydrogel.

12. The method according to any of claims 1 to 11, wherein the step d) is performed by first completely submerging the skin model in the suitable medium for at least 2 days and then culturing the cells comprised in the skin model in an air-liquid interface system for at least 10 days, preferably 14 days.

13. A biomimetic squamous cell carcinoma skin model obtained or obtainable from the method according to claims 1 to 12.

14. *In vitro* use of a biomimetic squamous cell carcinoma skin model obtained or obtainable from the method according to claims 1 to 12 in pharmacological development and preclinical screening of chemotherapeutic drugs, and basic study of the fundamental biology of cancer and the processes that mediate tumor proliferation, growth, metastasis and invasion.
